# EUROPEAN PATENT SPECIFICATION

(11) **EP 3 373 804 B1**
(45) Date of publication and mention of the grant of the patent: **26.05.2021**
(21) Application number: 16794642.5
(22) Date of filing: 11.11.2016
(51) Int. Cl.: A61B 5/0205, A61B 5/00, A61B 5/11, A61B 5/08

(54) **DEVICE, SYSTEM AND METHOD FOR SENSOR POSITION GUIDANCE**
VORRICHTUNG, SYSTEM UND VERFAHREN ZUR SENSORPOSITIONSFÜHRUNG
DISPOSITIF, SYSTÈME ET PROCÉDÉ DE GUIDAGE DE POSITION DE CAPTEUR

(30) Priority: 13.11.2015 US 201562255190 P; 07.12.2015 EP 15198149
(43) Date of publication of application: 19.09.2018
(73) Proprietor: Koninklijke Philips N.V., 5656 AG Eindhoven (NL)
(72) Inventor: GREGG, Richard, E., 5656 AE Eindhoven (NL); ATALLAH, Louis, Nicolas, 5656 AE Eindhoven (NL); MÜHLSTEFF, Jens, 5656 AE Eindhoven (NL); BONGERS, Edwin, Gerardus, Johannus, Maria, 5656 AE Eindhoven (NL)
(74) Representative: Philips Intellectual Property & Standards
(86) International application number: PCT/EP2016/077481
(87) International publication number: WO 2017/081284

(56) References cited:
- US-A1- 2008 214 963
- LUKAC TOMAS ET AL: "Contactless recognition of respiration phases using web camera", 2014 24TH INTERNATIONAL CONFERENCE RADIOELEKTRONIKA, IEEE, 15 April 2014 (2014-04-15), pages 1-4, XP032605668, DOI: 10.1109/RADIOELEK.2014.6828427 ISBN: 978-1-4799-3714-1 [retrieved on 2014-06-09]
- HUNG P D ET AL: "Estimation of respiratory waveform using an accelerometer", BIOMEDICAL IMAGING: FROM NANO TO MACRO, 2008. ISBI 2008. 5TH IEEE INTERNATIONAL SYMPOSIUM ON, IEEE, PISCATAWAY, NJ, USA, 14 May 2008 (2008-05-14), pages 1493-1496, XP031271335, ISBN: 978-1-4244-2002-5
- GHUFRAN SHAFIQ ET AL: "Surface Chest Motion Decomposition for Cardiovascular Monitoring", SCIENTIFIC REPORTS, vol. 4, 28 May 2014 (2014-05-28), XP055262217, DOI: 10.1038/srep05093

## Description

### FIELD OF THE INVENTION

The present invention relates to a device, system and method for sensor position guidance, in particular for guiding a subject (e.g. a patient, a nurse, more generally a person) or user to position a wearable sensor for monitoring of respiration and/or heart rate at the subject's body.

### BACKGROUND OF THE INVENTION

Wearable sensors are playing an important role in hospital and home care as well as consumer lifestyle (e.g. sports monitoring, child care, elderly care, etc.). However, many of these sensors are used at a fixed body location. Changing the location of the sensor provides in some cases a completely different signal due to the underlying physiology or sensitivity to artifacts.

Respiration rate, for instance, can be estimated using an accelerometer on the chest. Respiration is often monitored in sleep studies using more than one resistive or inductive belt because people breathe differently. Some people expand their ribs more, some people use their diaphragm more. Each person will have their own optimal accelerometer placement to maximize movement due to breathing.

Respiration can also be estimated from video. The motion in a person's chest can be estimated to determine the respiration rate. However, video based respiration monitoring alone will not work e.g. for an ambulatory patient.

T. Lukáč, J. Púčik and L. Chrenko, "Contactless recognition of respiration phases using web camera," Radioelektronika (RADIOELEKTRONIKA), 2014 24th International Conference, Bratislava, 2014, pp. 1-4 discloses a method for the extraction of respiration phases from a video sequence. A single-step Lucas-Kanade method for obtaining velocities is implemented and a signal to noise ratio of pixel blocks for a selection of the tracking blocks is calculated. ECG is simultaneously measured with web-camera recording and ECG derived respiration is compared with respiration derived by the disclosed method.

### SUMMARY OF THE INVENTION

It is an object of the present invention to provide a device, system and method that guide the subject or another user where to optimally place a wearable sensor for monitoring of respiration, and/or heart rate at the subject's body.

In a first aspect of the present invention, a device for sensor position guidance is presented as defined in claim 1.

In a further aspect of the present invention, a system for sensor position guidance is presented comprising
- a imaging unit for acquiring said image data of at least a subject's body area showing motion of said body area caused by respiration and/or heart beat, and
- the device for sensor position guidance based on the acquired image data.

In a yet further aspect of the present invention a corresponding method for sensor position guidance is presented.

In yet further aspects of the present invention, there are provided a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed system, method, computer program and medium have similar and/or identical preferred embodiments as the claimed device, in particular as defined in the dependent claims and as disclosed herein.

The present invention is based on the idea to use image data, such as video obtained by a video camera, to determine a point of maximum motion and use this point as the optimal position for placement of the wearable sensor at the subject's body. The image data hence should generally show motion caused by respiration and/or heart beat. Thus, the problems of the inability to follow a subject around with a video camera for respiration rate monitoring and of potentially poor lighting for video based monitoring of respiration are overcome. Further, optimal placement for a sensor based respiratory rate monitor for each of a variety of patients in a hospital or medical care center and, hence, optimal signal quality can be achieved. Thus, time consuming and error-prone trial and error placement of the sensor, as currently done to obtain the best signal, can be avoided and a personalized sensor placement tailored to the particular subject is achieved.

According to the present invention, said analyzer is configured to analyze the obtained image data by generating a motion map indicating the strength of motion in the body area and/or a pulsatility map indicating the strength of pulsatility in the body area and to determine the one or more locations of the body showing maximal movement caused by respiration and/or heart beat by use of the motion map and/or the pulsatility map. Algorithms for obtaining such motion maps and pulsatility maps are generally known in the art of image processing and vital signs monitoring.

Said guidance output is configured to select, as the optimum location, a point of maximal strength of motion in the motion map or a point of maximal strength of pulsatility in the pulsatility map. Essentially, a map of respiration-based motion and pulse-based motion are obtained. The optimum location can be found e.g. by weighting the pulse signal strength vs. respiration or by defining an SNR and search for an optimum location using a cost function. Also a body motion map measured or predefined can be included. The device may also indicate when a wearable sensor will not work.

The device may further comprise a subject data input for obtaining subject-related data comprising comfort levels for different locations of the subject's body indicating the subject's comfort and/or the possibility of placing a wearable sensor at the respective location. The subject-related data may e.g. be obtained from a hospital database or an electronic health record, for instance accessed via a network, such as the internet, LAN or WLAN.

Alternatively, the analyzer may be configured to determine, from the obtained image data, comfort levels for different locations of the subject's body indicating the subject's comfort and/or the possibility of placing a wearable sensor at the respective location.

The comfort level for a particular position of the subject's body may hereby indicate if (and optionally how much) it would be uncomfortable for the subject if a wearable sensor were placed at this position. For instance, if the subject has wounds or scars, it may be painful to place a sensor at such a position even if this may be the optimal position from the perspective of maximum movement. Further, the comfort level may indicate if (and optionally to which extent) it would be impossible to place a sensor at a particular position. For instance, if the subject wears a bandage it is not possible to place a sensor there, which can be indicated by the comfort level accordingly. Hence, an optimal position of the wearable sensor may be found as a trade-off between the maximum motion versus the sensitivity to artifacts and/or placement challenges (e.g. body shapes, bandages, wounds, etc.). According to the present invention, a separate map (i.e. a restrictions map) is used reflecting restrictions regarding the placement of the wearable sensor.

Preferably, said guidance output is configured to select from the determined one or more locations an optimum location based on the strength of movement and the comfort levels. Hence, in addition to the optimal placement of the sensor as explained above, other near optimal locations may be determined from which an optimum location is selected in situations where the sensor cannot be placed at the most optimum location (from the perspective of movement) due to bandages, skin breakdown or some other similar reasons reflected by the comfort levels. Optionally, a priori recommended body locations can be used in addition for determining the one or more locations.

In another embodiment the device further comprises a user interface for receiving said guidance information and for indicating the selected location to a user based on said guidance information. The user interface may e.g. comprise a display for indicating the selected location in image and/or text form. This helps the subject and/or a user (e.g. a nurse or care giver) to optimally place the sensor at the subject's body. The user interface may hereby be configured to show the selected location as a projection on an image of the subject's body so that it is easily visible where to place the sensor at this particular subject.

Still further, the device may comprise a vital sign determination unit for determining respiration and/or heart rate of the subject from the obtained image data, wherein said analyzer is configured to use the determined respiration and/or heart rate of the subject in the determination of the one or more locations of the body area showing maximal movement caused by respiration and/or heart beat. It is not given that the motion shown in the obtained image data is caused by breathing and heart beat (pulse) only, since e.g. wrinkles, shadows etc. can compromise the measurements. Therefore, respiration and/or pulse detection is obtained in this embodiment from image data of freely visible skin e.g. the forehead, the hand, the cheeks, etc. (detected automatically), preferably acquired with the same imaging unit. For this purpose the known principle of remote photoplethysmography (PPG) can be applied. The obtained respiration and/or heart rate is then used, as a kind of cross-check, if the motion detected in the images is caused by respiration and/or heart beat, e.g. by deriving respiration and/or heart rate from said motion and comparing them with the respiration and/or heart rate derived from the image data by use of remote PPG.

In still another embodiment other reference signals for respiration and/or heart rate, as e.g. acquired by use of separate respiration and/or heart rate sensors, can be used for this purpose.

Still further, in an embodiment the user interface may be used to guide the user to breathe in a predetermined way, e.g. with a predetermined respiration rate. In this case the respiration rate is known, which can thus be used in the above described check if the motion in the image data is caused by respiration or not.

As mentioned above, the image data are acquired by an imaging unit, which may comprise a camera (e.g. a video camera, RGB camera, web cam, etc.), a range camera, a radar device or a scanner (e.g. a laser scanner).

In an embodiment the imaging unit is configured to acquire image data while the subject or a user is placing the sensor at the selected location at the subject's body and an image processor is provided for checking, based on the acquired image data, if the sensor is placed at the correct selected position. If this is not the case a feedback may immediately be given to the subject and/or user to correct the location of the sensor.

The system may further comprise a projection unit for projecting an indicator at the selected location onto the subject's body. The projection unit may e.g. be a laser pointer, light source or beamer, and the indicator may simply be a small spot or other sign (e.g. an arrow or cross) at the location where the wearable sensor should be positioned.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an embodiment of a system and device for sensor position guidance according to the present invention,
Fig. 2 shows a flow chart of an embodiment of a method for sensor position guidance according to the present invention, and
Fig. 3 shows a schematic diagram of another embodiment of a system and device for sensor position guidance according to the present invention.

### DETAILED DESCRIPTION OF THE INVENTION

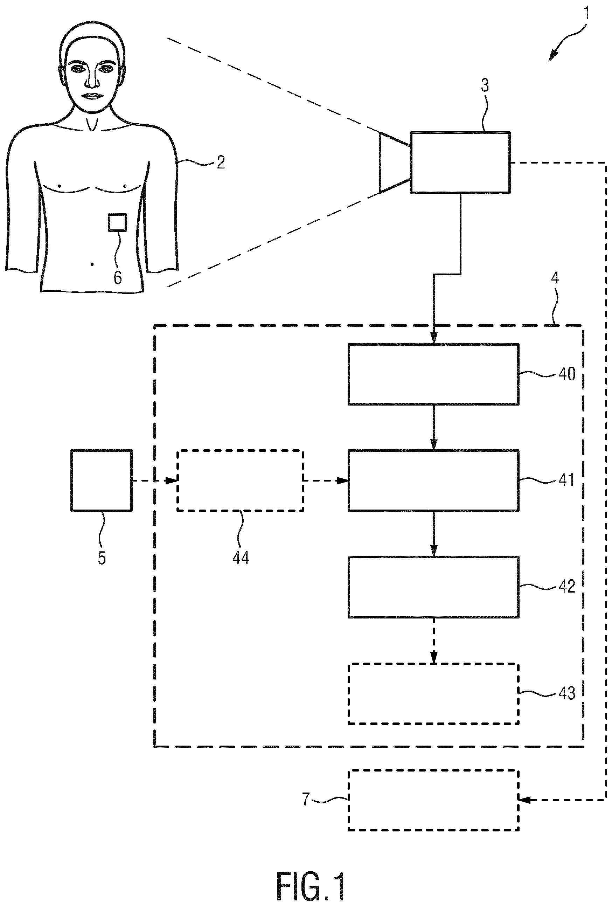

Fig. 1 shows a schematic diagram of an embodiment of a system 1 and device 4 for sensor position guidance according to the present invention for guiding a subject 2, such as a patient, elderly person or athlete, or a user, such as a nurse or caregiver, to place a wearable sensor 6, in particular for heart rate and/or respiration measurements, at the best possible position at the subject's body. The wearable sensor 6 may e.g. be an acceleration sensor (or accelerometer) for monitoring movements (preferably in three different dimensions) of the subject's chest wall or abdominal wall caused by respiration and/or heart beat, which measurements allow deriving vital signs of the subject like respiration rate and heart rate. Another example for such a wearable sensor is a gyroscope or other motion sensitive device, which may also be embedded into another multi-functional device that shall be mounted to the subject's body. Still another example of a wearable sensor allowing heart rate and respiration rate estimation is a sensor for reflectance pulse oximetry which measures, roughly, color change of the skin due to flushing by oxygenated blood. The wearable sensor 6 may e.g. be mounted to the subject's body by use of an adhesive tape or a belt.

The system 1 comprises an imaging unit 3 for acquiring image data of at least a subject's body area (e.g. of the subject's torso) showing motion of said body area caused by respiration and/or heart beat. The imaging unit 3 may e.g. include a camera, a radar device or a scanner (e.g. a laser scanner), which is able to detect image data from which the desired movements can be extracted. The system 1 further comprises the device 4 for sensor position guidance based on the acquired image data. This device 4 may be implemented in hardware, software or a mixture of hard- and software, for instance as software running on a processor or computer, which may also be embedded into another device. For instance, corresponding software for implementing the proposed device 4 may be provided, e.g. as application program ("app"), on a user device, such as a smartphone, smart watch or pulse oximeter.

The device 4 comprises an image data input 40 for obtaining image data of at least a subject's body area showing motion of said body area caused by respiration and/or heart beat. The image data may directly be provided (e.g. transmitted, in a wireless or wired manner) from the imaging unit 3, or may be received or fetched (e.g. downloaded) from a storage or buffer. The image data input 40 may thus be implemented as a data interface for receiving or retrieving image data.

The device 40 further comprises an analyzer 41 for analyzing the obtained image data to determine one or more locations of the body area showing maximal movement caused by respiration and/or heart beat. For this purpose the analyzer 41 may use a known algorithm for motion detection in image data. Exemplary methods include algorithms that perform background subtraction, or perform foreground motion detection by difference-based spatial temporal entropy image, or compare an incoming video image to a reference image. A point of maximum movement could hereby be maximum displacement or maximum velocity, depending on which gives the best movement signal (or derived vital signs signal, such as respiratory signal or heart rate signal) to noise ratio. Additional exemplary methods include a range camera using light coding technology like a camera as used in various gaming systems.

The device 40 further comprises a guidance output 42 for selecting from the determined one or more locations an optimum location based on the strength of movement. Further, it provides guidance information indicating the optimum location, at which the wearable sensor 6 should be placed to the subject's body. The guidance information may be information sent to another (external) entity, e.g. a hospital computer or a device used by nurse or a patient monitor, where the information may be further processed, e.g. to be output as instructions for the user instructing the user where to place the wearable sensor 6. The guidance information may also be provided for being retrieved if desired by said another entity.

The result of placing the wearable sensor 6 at a point of maximal movement is an improved signal to noise ratio (SNR) in the movement signal as well as in the derived vital signs signal, resulting finally in a higher accuracy in the estimated vital signs signal, such as the estimated respiration rate and/or heart rate, e.g. due to reduced sensitivity to motion artefacts.

In an advantageous embodiment the device 40 may comprise a user interface 43, such as a display and/or loudspeaker, for receiving said guidance information and for indicating the determined location to a user based on said guidance information. The user interface 43 may for instance comprise a display, on which the determined location is indicated in image and/or text form. The determined location may e.g. be shown on the display as a projection on an image of the subject's body. For instance, an image of the real subject may be shown as an image, in which, e.g. by an arrow or as a graphical symbol, the desired position for the sensor is indicated. Alternatively, a general (e.g. graphical) image of a body may be shown in which the position is indicated or an indicator or even an image of the wearable sensor 6 may be projected onto the selected location on the patient's body, e.g. by a light or laser projector.

The device may further optionally comprise a subject data input 44 for obtaining subject-related data comprising comfort levels for different locations of the subject's body indicating the subject's comfort and/or the possibility of placing a wearable sensor at the respective location. The subject data input 44 may be an interface to receive or retrieve such subject-related data, e.g. from a central database 5 of a hospital or an electronic health record or generally any look-up table. The subject data input 44 may also be a kind of user interface allowing the user and/or the subject to enter subject-related data. Such subject related data may e.g. be information where a wearable sensor should or even could not be placed at the subject's body, e.g. because of wounds or scars or a bandage or another medical device that is placed somewhere at the subject's body. In addition, a level of comfort (indicating how comfortable or uncomfortable it would be if a wearable sensor were placed at the respective position) may be provided.

The analyzer 41 is configured to determine, from the obtained image data, such restrictions in sensor placement. For instance, places where the subject has wounds, scars, bandages or other equipment can generally be detected by use of image processing methods.

In both cases the guidance output 42 then takes the strength of movement and, additionally, the placement restrictions into account when selecting, from the determined one or more locations, an optimum location for placement of the wearable sensor. The thus selected "optimum" solution may then be a sub-optimum solution from the perspective of movements, but it still provides an advantage over the conventional methods. It may also happen that the measured motion is too low for the wearable sensor and the use of the sensor for a particular patient is not advised.

In another embodiment the imaging unit 3 is configured to acquire image data while the subject 2 or a user is placing the sensor 6 at the selected location at the subject's body. These image data are provided to an additional image processor7 which checks, based on the acquired image data, if the sensor 6 is placed at the correct selected position. If it is detected that the sensor 6 is detected at a wrong position a corresponding feedback, e.g. a text notice or a sound signal via the user interface 43, may immediately be given to the subject and/or user to correct the location of the sensor 6. The image processor 7 may hereby be part of the device 4 or may be a separate entity.

In still another embodiment the analyzer 41 may analyze the obtained image data by generating a motion map indicating the strength of motion in the body area and/or a pulsatility map indicating the strength of pulsatility in the body area. The motion map and/or the pulsatility map may then be used to determine the one or more locations of the body showing maximal movement caused by respiration and/or heart beat. Further, the guidance output 42 may select, as the optimum location, a point of maximal strength of motion in the motion map or a point of maximal strength of pulsatility in the pulsatility map. A motion map generally represents the position, velocity, and acceleration of an object (e.g. an image feature, at various moments in time. A pulsatility map measures blood volume changes cause by heart beat, wherein pulsatility is defined as the AC/DC component of an acquired light absorption signal.

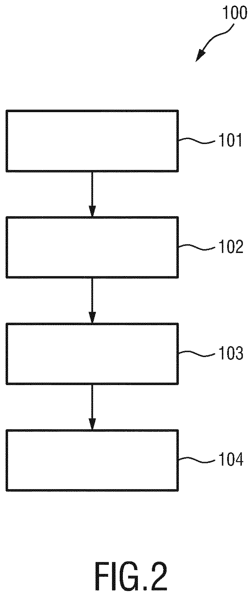

Fig. 2 shows a flow chart of an embodiment of a method 100 for sensor position guidance according to the present invention. In a first step 101, image data of at least a subject's body area showing motion of said body area caused by respiration and/or heart beat are obtained. In a second step 102, the obtained image data are analyzed to determine one or more locations of the body area showing maximal movement caused by respiration and/or heart beat. In a third step 103, from the determined one or more locations an optimum location based on the strength of movement is selected. In a fourth step 104, guidance information indicating the optimum location, at which a wearable sensor for monitoring respiration and/or heart rate should be placed to said subject's body, is provided.

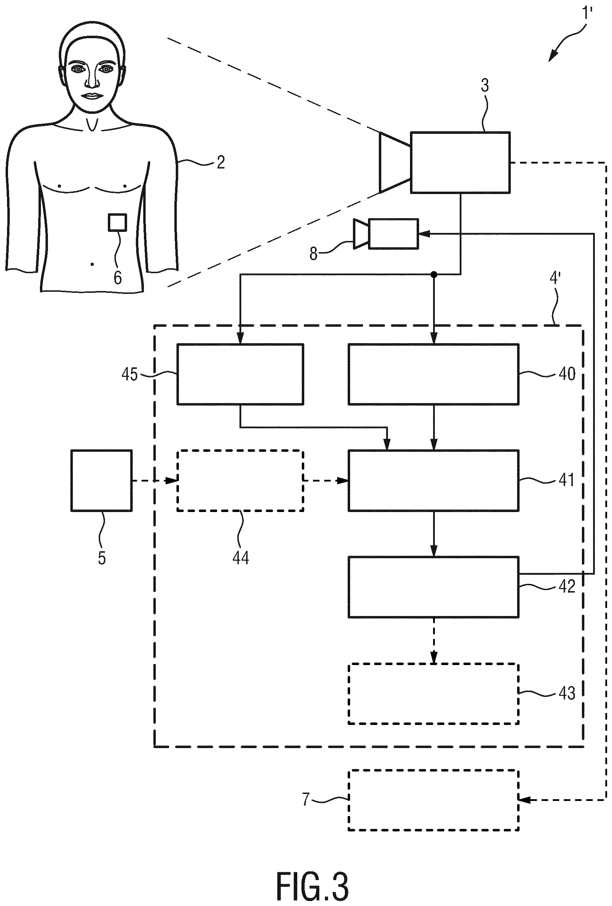

Fig. 3 shows a schematic diagram of another embodiment of a system 1' and device 4' for sensor position guidance according to the present invention, which comprise additional optional elements, which may also be used separately and in other combinations.

In this embodiment, the system 1' further comprises a projection unit 8. Based on the guidance information the projection unit 8, e.g. a laser pointer or other projector, for projects an indicator (e.g. a light spot, a cross, an arrow, an image of the wearable sensor or any other sign) at the selected location onto the subject's body. For this purpose, the projection direction of the projection unit 8 is adjustable and the current position of the subject's body should be known, which can be derived from the images obtained by the imaging unit 3. The subject 2 or a user can this directly see where the wearable sensor should optimally be placed. A laser projector could project a cross hair or a line drawing type of representation of the wearable sensor. The user can hence look at the subject and not at image of the subj ect.

Further, device 4' comprises a vital sign determination unit 45 for determining respiration and/or heart rate of the subject from the obtained image data, e.g. by use of remote photoplethysmography (remote PPG) as e.g. described in Verkruysse et al., "Remote plethysmographic imaging using ambient light", Optics Express, 16(26), 22 December 2008, pp. 21434-21445 or many other documents. The determined respiration and/or heart rate of the subject is then used by the analyzer for the determination of the one or more locations of the body area showing maximal movement caused by respiration and/or heart beat. In particular, it is checked if respiration and/or heart rate derived by an analysis of said motion corresponds to respiration and/or heart rate derived from the image data by use of remote PPG. If the rates are similar or identical it is assumed that the motion is caused by respiration and/or heart rate, and otherwise not.

Instead of deriving the respiration and/or heart rate another reference signal may be obtained, e.g. from a separate sensor (e.g. a pulse oximeter, a respiration sensor, etc.; not shown) mounted to the subject's body. This reference signal can then also be used by the analysis unit in the same way as explained above for checking if the motion is caused by respiration and/or heart beat.

Still further, in an embodiment the user interface 43 may be used to provide a respiration guidance signal to guide the user to breathe in a predetermined way, e.g. with a predetermined respiration rate. In this case the respiration rate is known, which can thus be used in the above described check by the analysis unit 41 if the motion in the image data is caused by respiration or not.

The present invention can be applied in many different scenarios where wearable sensor shall be mounted to a subject's body. It may e.g. help a nurse or healthcare professional to place an accelerometer based respiration monitor at the best possible location on a patient's chest or abdomen being personalized for a patient's specific situation. This will provide the best signal and potentially reduce false alarms due to missing or inaccurate respiration rate. Further areas of application include perioperative scenarios, intensive care units and monitoring patients in the ward.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims, which define the invention.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A device for sensor position guidance, said device comprising:
- an image data input (40) configured to obtain image data of at least a subject's body area showing motion of said body area,
- an analyzer (41) configured to analyze the obtained image data to generate a motion map indicating the strength of motion in the body area and/or a pulsatility map indicating the strength of pulsatility in the body area and to determine, by use of the motion map and/or the pulsatility map, one or more locations of the body area showing maximal movement caused by respiration and/or heart beat based on the strength of motion and/or the strength of pulsatility in the body area, as well as
a restrictions map indicating placement restrictions, wherein places where the subject has wounds, scars, skin breakdown, bandages or other equipment are subject to the placement restrictions, and
- a guidance output (42) configured to select from the determined one or more locations not subject to the placement restrictions, an optimum location, at which a wearable sensor (6) for monitoring respiration and/or heart rate should be placed to said subject's body, based on the strength of movement by selecting, as the optimal location, a point of maximal strength of motion in the motion map or a point of maximal strength of pulsatility in the pulsatility map, and to provide guidance information indicating the optimum location.

2. The device as claimed in claim 1,
further comprising a subject data input (44) configured to obtain subject-related data comprising comfort levels for different locations of the subject's body indicating the subject's comfort and/or the possibility of placing a wearable sensor at the respective location.

3. The device as claimed in claim 1,
wherein said analyzer (41) is configured to determine, from the obtained image data, comfort levels for different locations of the subject's body indicating the subject's comfort and/or the possibility of placing a wearable sensor at the respective location.

4. The device as claimed in claim 2 or 3,
wherein said guidance output (42) is configured to select from the determined one or more locations an optimum location based on the strength of movement and the comfort levels.

5. The device as claimed in claim 1,
further comprising a user interface (43) configured to receive said guidance information and for indicating the selected location to a user based on said guidance information.

6. The device as claimed in claim 5,
wherein said user interface (43) comprises a display configured to indicate the selected location in image and/or text form, in particular to show the selected location as a projection on an image of the subject's body.

7. The device as claimed in claim 6,
further comprising a vital sign determination unit (45) configured to determine respiration and/or heart rate of the subject from the obtained image data, wherein said analyzer (41) is configured to use the determined respiration and/or heart rate of the subject in the determination of the one or more locations of the body area showing maximal movement caused by respiration and/or heart beat.

8. A system for sensor position guidance, said system comprising
- a imaging unit (3) configured to acquire said image data of at least a subject's body area showing motion of said body area, and
- a device (4) as claimed in any of the preceding claims for sensor position guidance based on the acquired image data.

9. The system as claimed in claim 8,
wherein said imaging unit (3) comprises a camera, a range camera, a radar device or a scanner.

10. The system as claimed in claim 8,
wherein the imaging unit (3) is configured to acquire image data while the subject or a user is placing the sensor at the selected location at the subject's body and wherein the system further comprises an image processor (7) for checking, based on the acquired image data, if the sensor is placed at the correct selected position.

11. The system as claimed in claim 8,
further comprising a projection unit (8) configured to project an indicator at the selected location onto the subject's body.

12. A method for sensor position guidance, said method comprising:
- obtaining image data of at least a subject's body area showing motion of said body area,
- analyzing the obtained image data to generate a motion map indicating the strength of motion in the body area and/or a pulsatility map indicating the strength of pulsatility in the body area, as well as a restrictions map indicating placement restrictions, wherein places where the subject has wounds, scars, skin breakdown, bandages or other equipment are subject to the placement restrictions,
- determining one or more locations of the body area showing maximal movement caused by respiration and/or heart beat based on the strength of motion and/or the strength of pulsatility in the body area,
- selecting from the determined one or more locations not subject to the placement restrictions, an optimum location, at which a wearable sensor (6) for monitoring respiration and/or heart rate should be placed to said subject's body, based on the strength of movement by selecting, as the optimal location, a point of maximal strength of motion in the motion map or a point of maximal strength of pulsatility in the pulsatility map, and
- providing guidance information indicating the optimum location.

13. Computer program comprising program code means for causing a computer to carry out the steps of the method as claimed in claim 12 when said computer program is carried out on the computer.

## Patentansprüche

1. Ein Gerät für die Sensorpositionsführung, wobei das Gerät Folgendes umfasst:
- einen Bilddateneingang (40), um Bilddaten von mindestens einer Körperregion eines Patienten zu erhalten, die eine Bewegung der Körperregion angeben,
- eine Analyseeinheit (41), die die abgerufenen Bilddaten analysiert, um eine Bewegungskarte, die die Bewegungsstärke in der Körperregion angibt, und/oder eine pulsatile Sekretionskarte zu erstellen, die die Stärke der pulsatilen Sekretion in der Körperregion angibt, und um mithilfe der Bewegungskarte und/oder der pulsatilen Sekretionskarte mindestens eine Stelle der Körperregion zu ermitteln, die beruhend auf der Stärke der Bewegung und/oder der pulsatilen Sekretion in der Körperregion eine durch Atmung und/oder Herzschlag verursachte maximale Bewegung aufweist, sowie
eine Einschränkungskarte, die Platzierungseinschränkungen angibt, wobei Stellen, an denen der Patient Wunden, Narben, Hautrisse, Verbände oder andere Vorrichtungen aufweist, den Platzierungseinschränkungen unterliegen, und
- einen Führungsausgang (42), der unter der mindestens einen ermittelten, nicht den Platzierungseinschränkungen unterliegenden Stelle eine optimale Stelle auswählt, an der ein tragbarer Sensor (6) zum Überwachen der Atmung und/oder der Herzfrequenz am Körper des Patienten platziert werden sollte. Dies erfolgt anhand der Bewegungsstärke, indem in der Bewegungskarte als optimale Stelle ein Punkt mit maximaler Bewegungsstärke oder in der pulsatilen Sekretionskarte ein Punkt mit maximaler pulsatiler Sekretionsstärke ausgewählt und Führungsdaten bereitstellt werden, die die optimale Stelle angeben.

2. Das Gerät gemäß Anspruch 1,
das zudem einen Patientendateneingang (44) umfasst, um patientenbezogene Daten abzurufen, die die Komfortniveaus verschiedener Stellen am Körper des Patienten umfassen, die den Komfort des Patienten und/oder die Möglichkeit der Platzierung eines tragbaren Sensors an der jeweiligen Stelle angeben.

3. Das Gerät gemäß Anspruch 1,
wobei die Analyseeinheit (41) anhand der abgerufenen Bilddaten Komfortniveaus für verschiedene Stellen des Körpers des Patienten ermittelt, die den Komfort des Patienten und/oder die Möglichkeit der Platzierung eines tragbaren Sensors an der jeweiligen Stelle angeben.

4. Das Gerät gemäß Anspruch 2 oder 3,
wobei der Führungsausgang (42) beruhend auf der Bewegungsstärke oder den Komfortniveaus unter der mindestens einen ermittelten Stelle eine optimale Stelle auswählt.

5. Das Gerät gemäß Anspruch 1,
das zudem eine Benutzerschnittstelle (43) umfasst, die die Führungsdaten abruft und dem Benutzer die anhand der Führungsdaten ausgewählte Stelle anzeigt.

6. Das Gerät gemäß Anspruch 5,
wobei die Benutzerschnittstelle (43) eine Anzeige umfasst, die die ausgewählte Stelle in Bild- und/oder Textform anzeigt, und die insbesondere die ausgewählte Stelle als Projektion auf einem Bild des Körpers des Patienten anzeigt.

7. Das Gerät gemäß Anspruch 6,
das zudem eine Vitalparameter-Ermittlungseinheit (45) umfasst, die die Atmung und/oder die Herzfrequenz des Patienten anhand der abgerufenen Bilddaten ermittelt, wobei die Analyseeinheit (41) die ermittelte Atmung und/oder Herzfrequenz des Patienten beim Ermitteln der mindestens einen Stelle am Körper verwendet, die eine durch Atmung und/oder Herzschlag verursachte maximale Bewegung aufweist.

8. Ein System für die Sensorpositionsführung, wobei das System Folgendes umfasst:
- eine Bildgebungseinheit (3), um die Bilddaten von mindestens einer Körperregion eines Patienten abzurufen, die eine Bewegung der Körperregion angeben, und
- ein Gerät (4) gemäß einem der vorherigen Ansprüche für die Sensorpositionsführung anhand der abgerufenen Bilddaten.

9. Das System gemäß Anspruch 8,
wobei die Bildgebungseinheit (3) eine Kamera, eine Distanzbildkamera, ein Radargerät oder einen Scanner umfasst.

10. Das System gemäß Anspruch 8,
wobei die Bildgebungseinheit (3) Bilddaten erfasst, während der Patient oder ein Benutzer den Sensor an der ausgewählten Stelle am Körper des Patienten platziert, und wobei das System zudem einen Bildprozessor (7) umfasst, um anhand der erfassten Bilddaten zu prüfen, ob der Sensor an der richtigen ausgewählten Position platziert wurde.

11. Das System gemäß Anspruch 8,
das zudem eine Projektionseinheit (8) umfasst, die eine Markierung auf die ausgewählte Stelle am Körper des Patienten projiziert.

12. Ein Methode für die Sensorpositionsführung, wobei die Methode folgende Schritte umfasst:
- Abrufen von Bilddaten von mindestens einer Körperregion eines Patienten, die eine Bewegung der Körperregion angeben,
- Analysieren der abgerufenen Bilddaten, um eine Bewegungskarte, die die Bewegungsstärke in der Körperregion angibt, und/oder eine pulsatile Sekretionskarte zu erstellen, die die Stärke der pulsatilen Sekretion in der Körperregion angibt, sowie einer Einschränkungskarte, die Platzierungseinschränkungen angibt, wobei Stellen, an denen der Patient Wunden, Narben, Hautrisse, Verbände oder andere Vorrichtungen aufweist, den Platzierungseinschränkungen unterliegen,
- beruhend auf der Stärke der Bewegung und/oder der pulsatilen Sekretion in der Körperregion Ermitteln mindestens einer Stelle am Körper, die eine durch Atmung und/oder den Herzschlag verursachte maximale Bewegung aufweist,
- Auswählen unter der mindestens einen ermittelten, nicht den Platzierungseinschränkungen unterliegenden Stelle einer optimalen Stelle, an der ein tragbarer Sensor (6) zum Überwachen der Atmung und/oder der Herzfrequenz am Körper des Patienten platziert werden sollte. Dies erfolgt anhand der Bewegungsstärke, indem in der Bewegungskarte als optimale Stelle ein Punkt mit maximaler Bewegungsstärke oder in der pulsatilen Sekretionskarte ein Punkt mit maximaler pulsatiler Sekretionsstärke ausgewählt wird, und
- Bereitstellen von Führungsdaten, die die optimale Stelle angeben.

13. Ein Computerprogramm, das Programmcode umfasst, mit dem ein Computer veranlasst wird, die Schritte der Methode gemäß Anspruch 12 auszuführen, wenn das Computerprogramm auf einem Computer ausgeführt wird.

## Revendications

1. Dispositif de guidage de la position du capteur, ledit dispositif comprenant :
- une entrée de données d'image (40) configurée pour obtenir des données d'image d'au moins une zone corporelle d'un sujet montrant le mouvement de ladite zone corporelle,
- un analyseur (41) configuré pour analyser les données d'image obtenues pour générer une carte de mouvement indiquant la force du mouvement dans la zone corporelle et/ou une carte de pulsatilité indiquant la force de la pulsatilité dans la zone corporelle et pour déterminer, en fonction de la carte de mouvement et/ou de la carte de pulsatilité, au moins un emplacement de la zone corporelle montrant le mouvement maximal causé par la respiration et/ou le rythme cardiaque en fonction de la force du mouvement et/ou de la force de la pulsatilité dans la zone corporelle, ainsi que
une carte de restrictions indiquant les restrictions de placement, dans lequel les emplacements où le sujet présente des blessures, des cicatrices, des lésions cutanées, des bandages ou d'autres équipements sont soumis aux restrictions de placement, et
- une sortie de guidage (42) configurée pour sélectionner parmi l'au moins un emplacement déterminé non soumis aux restrictions de placement, un emplacement optimal, auquel un capteur (6) portable pour surveiller la respiration et/ou la fréquence cardiaque doit être placé sur le corps dudit sujet, en fonction de la force du mouvement par sélection, comme emplacement optimal, d'un point de force maximale de mouvement dans la carte de mouvement ou d'un point de force maximale de pulsatilité dans la carte de pulsatilité, et pour fournir des informations de guidage indiquant l'emplacement optimal.

2. Dispositif selon la revendication 1,
comprenant en outre une entrée de données sujet (44) configurée pour obtenir des données relatives au sujet comprenant des niveaux de confort pour différents emplacements du corps du sujet indiquant le confort du sujet et/ou la possibilité de placer un capteur portable à l'emplacement respectif.

3. Dispositif selon la revendication 1,
dans lequel ledit analyseur (41) est configuré pour déterminer, en fonction des données d'image obtenues, des niveaux de confort pour différents emplacements du corps du sujet indiquant le confort du sujet et/ou la possibilité de placer un capteur portable à l'emplacement respectif.

4. Dispositif selon la revendication 2 ou 3,
dans lequel ladite sortie de guidage (42) est configurée pour sélectionner parmi l'au moins un emplacement déterminé un emplacement optimal en fonction de la force du mouvement et des niveaux de confort.

5. Dispositif selon la revendication 1,
comprenant en outre une interface utilisateur (43) configurée pour recevoir lesdites informations de guidage et pour indiquer l'emplacement sélectionné à un utilisateur en fonction desdites informations de guidage.

6. Dispositif selon la revendication 5,
dans lequel ladite interface utilisateur (43) comprend un affichage configuré pour indiquer l'emplacement sélectionné sous forme d'image et/ou de texte, en particulier pour montrer l'emplacement sélectionné sous forme de projection sur une image du corps du sujet.

7. Dispositif selon la revendication 6,
comprenant en outre une unité de détermination des signes vitaux (45) configurée pour déterminer la respiration et/ou la fréquence cardiaque du sujet en fonction des données d'image obtenues, dans lequel ledit analyseur (41) est configuré pour utiliser la respiration et/ou la fréquence cardiaque déterminées du sujet dans la détermination de l'au moins un emplacement de la zone corporelle montrant un mouvement maximal provoqué par la respiration et/ou le rythme cardiaque.

8. Système de guidage de la position du capteur, ledit système comprenant
- une unité d'imagerie (3) configurée pour acquérir lesdites données d'image d'au moins une zone corporelle d'un sujet montrant le mouvement de ladite zone corporelle, et
- un dispositif (4) selon l'une quelconque des revendications précédentes destiné au guidage de la position du capteur en fonction des données d'image acquises.

9. Système selon la revendication 8,
dans lequel ladite unité d'imagerie (3) comprend une caméra, une caméra de distance, un dispositif radar ou un balayeur.

10. Système selon la revendication 8,
dans lequel l'unité d'imagerie (3) est configurée pour acquérir des données d'image pendant que le sujet ou un utilisateur place le capteur à l'emplacement sélectionné sur le corps du sujet, et dans lequel ledit système comprend en outre un processeur d'image (7) destiné à la vérification, en fonction des données d'image acquises, si le capteur est placé à la position sélectionnée correcte.

11. Système selon la revendication 8,
comprenant en outre une unité de projection (8) configurée pour projeter un indicateur à l'emplacement sélectionné sur le corps du sujet.

12. Procédé de guidage de la position du capteur, ledit procédé comprenant:
- l'obtention des données d'image d'au moins une zone corporelle d'un sujet montrant le mouvement de ladite zone corporelle,
- l'analyse des données d'image obtenues pour générer une carte de mouvement indiquant la force du mouvement dans la zone corporelle et/ou une carte de pulsatilité indiquant la force de la pulsatilité dans la zone corporelle, ainsi qu'une carte de restrictions indiquant les restrictions de placement, dans lequel les emplacements où le sujet présente des blessures, des cicatrices, des lésions cutanées, des bandages ou d'autres équipements sont soumis aux restrictions de placement,
- la détermination d'au moins un emplacement de la zone corporelle montrant un mouvement maximal provoqué par la respiration et/ou le rythme cardiaque en fonction de la force du mouvement et/ou de la force de la pulsatilité dans la zone corporelle,
- la sélection parmi l'au moins un emplacement déterminé non soumis aux restrictions de placement, un emplacement optimal, auquel un capteur portable (6) doit être placé pour surveiller la respiration et/ou la fréquence cardiaque sur le corps dudit sujet, en fonction de la force du mouvement par sélection, comme emplacement optimal, d'un point de force maximale de mouvement dans la carte de mouvement ou d'un point de force maximale de pulsatilité dans la carte de pulsatilité, et
- la fourniture des informations de guidage indiquant l'emplacement optimal.

13. Programme informatique comprenant un moyen de code de programme permettant d'amener un ordinateur à mettre en œuvre les étapes du procédé selon la revendication 12 lorsque ledit programme d'ordinateur est exécuté sur l'ordinateur.
